# FASCICULE DE BREVET EUROPEEN

(11) **EP 0 333 546 B1**
(45) Date de publication et mention de la délivrance du brevet: **28.12.1994**
(21) Numéro de dépôt: 89400588.3
(22) Date de dépôt: 02.03.1989
(51) Int. Cl.: A61B 17/58

(54) **Tuyau intra médullaire pour empêcher la rotation des fragments d'un os long fracturé**
Intramedulläres Rohr zum Verhindern der Umdrehung von Splittern eines gebrochenen langen Knochens
Intramedullary tube for preventing the rotation of the fragments of a long fractured bone

(30) Priorité: 03.03.1988 FR 8802709
(43) Date de publication de la demande: 20.09.1989
(73) Titulaire: Loutfi, Rachid, F-95300 Pontoise (FR)
(72) Inventeur: Loutfi, Rachid, F-95300 Pontoise (FR)
(74) Mandataire: Rinuy, Santarelli

(56) Documents cités:
- EP-A- 0 118 778
- DE-B- 2 260 839
- DE-C- 745 873
- FR-A- 1 416 585
- FR-A- 2 141 020
- FR-A- 2 484 243
- US-A- 4 204 531
- US-A- 4 275 717
- US-A- 4 475 545
- US-A- 4 622 959

## Description

La présente invention concerne un tuyau intra-médullaire pour stabiliser la fracture d'un os long en empêchant la rotation des fragments l'un par rapport à l'autre.

La plupart des éléments de soutien en forme de tuyau n'empêchent pas de façon stable la rotation des fragments d'un os fracturé et ceux de ce genre qui empêchent cette rotation nécessitent une longue durée d'intervention chirurgicale et une utilisation excessive de rayons X, ce qui comporte des inconvénients pour le patient, le chirurgien et les auxiliaires opératoires.

La demande de brevet européen N° 0.118.778 et le brevet US N° 4.475.545 décrivent par exemple un tuyau intra-médullaire qui nécessite une fixation par cloutage des deux parties de l'os fracturé.

Pour éviter ces perforations supplémentaires par rapport à celles permettant l'insertion du support dans le canal médullaire, le brevet français N° 2.141.020 et le brevet US N° 4.275.517 ont proposé des solutions complexes nécessitant un appareillage comportant plusieurs pièces.

Toujours en vue d'obtenir une stabilisation de la fracture, le brevet français N° 2.484.243 a proposé un élément de soutien composé de deux tuyaux concentriques.

Egalement en vue d'obtenir une telle stabilisation FR-A1 416 585 décrit un clou médullaire pour empêcher la rotation dans les fractures d'os longs comportant un dispositif destiné à empêcher la rotation des fragments de l'os fracturé l'un par rapport à l'autre à sa partie distale et à sa partie proximale, constitué d'un tuyau présentant un passage longitudinal et un passage transversal pour une tige destinée à empêcher la rotation du côté proximal et des lamelles, comportant une olive capable d'écarter lesdites lamelles grâce à un mandrin d'actionnement restant à demeure dans le tuyau implanté.

La présente invention s'est fixé pour but de réaliser un élément de soutien simple à réaliser, d'utilisation aisée, tout en étant doté d'une parfaite efficacité.

C'est pourquoi la présente invention a pour objet un clou intra-médullaire pour empêcher la rotation dans les fractures d'os longs comportant un dispositif destiné à empêcher la rotation des fragments de l'os fracturé l'un par rapport à l'autre à sa partie distale et à sa partie proximale, constitué d'un tuyau présentant un passage longitudinal et un passage transversal pour une tige destinée à empêcher la rotation du côté proximal et deux lamelles (2,2') prévues à l'extrémité distale, caractérisé en ce que les lamelles sont élastiques, spontanément écartées l'une de l'autre, et munies à proximité de leur extrémité distale d'éléments (3,3') perforés d'un canal (4,4') selon l'axe du tuyau et disposés vers l'intérieur du tuyau, éléments dans lesquels en passant par le passage longitudinal on introduit pour l'installation dans l'os long un guide (7) rapprochant les lamelles, et libérant celles-ci, qui s'écartent, lorsque le guide est retiré hors du clou.

Par "tuyau intra-médullaire", on entend un corps sensiblement cylindrique creux et allongé destiné à être introduit dans le canal médullaire d'un os long.

L'élément de soutien selon l'invention est très simple puisqu'il ne comporte que deux pièces : le tuyau qui par ses qualités propres bloque la partie distale de l'os et la tige perforant ledit tuyau et l'os, destinée à empêcher la rotation du côté proximal.

Dans des conditions préférentielles de mise en oeuvre, le tuyau intra-médullaire tel que défini ci-dessus est caractérisé en ce que le dispositif destiné à empêcher la rotation des fragments de l'os fracturé l'un par rapport à l'autre du côté distal est constitué de deux lamelles spontanément écartées l'une de l'autre.

Ces lamelles élastiques sont de préférence munies près de leur extrémité distale et à l'intérieur des tuyaux de bossages perforés longitudinalement de manière à permettre le passage d'un guide dans les perforations lorsque les lamelles sont rapprochées.

Afin de réaliser le blocage du côté proximal, le tuyau peut coopérer avec une tige perforant à la fois ledit tuyau et l'os.

Dans des conditions préférentielles de mise en oeuvre, ladite tige fait un angle d'environ 55° avec l'axe longitudinal du tuyau. La tige en question peut être par exemple un simple clou mais est de préférence une vis. Dans ce dernier cas, le passage de la tige est avantageusement fileté pour permettre une parfaite coopération.

Dans d'autres conditions préférentielles de mise en oeuvre de l'invention ci-dessus décrite, le tuyau est fendu sur environ la moitié de sa longueur. Bien entendu, cette fente ne doit pas altérer les qualités du tuyau intra-médullaire selon l'invention, c'est à dire sa rigidité en torsion et l'élasticité des lamelles.

Le tuyau a une forme avantageusement adaptée, par exemple courbe, de manière à épouser la forme du canal médullaire.

Le tuyau intra-médullaire et la tige sont réalisés dans les métaux et alliages habituels des prothèses.

Comme déjà indiqué, le tuyau intra-médullaire selon la présente invention est d'une utilisation remarquablement aisée. Tout d'abord, l'os à consolider est perforé longitudinalement afin de créer un passage dans le canal médullaire. On introduit dans le canal médullaire le guide dont la longueur est supérieure à celle de l'os. On rapproche les lamelles élastiques de manière que le guide introduit dans les perforations des bossages maintienne lesdites lamelles rapprochées lors du passage du tuyau dans la longueur du canal médullaire.

Une fois le tuyau arrivé à la partie élargie de l'extrémité distale de l'os, on retire le guide en le tirant simplement de manière que les lamelles s'écartent spontanément et bloquent l'os du fait de leur élasticité. Enfin, on continue d'enfoncer le tuyau jusqu'à ce que les lamelles puissent bien s'ancrer dans la partie spongieuse de l'extrémité distale de l'os.

La rotation du fragment distal de l'os fracturé est ainsi automatiquement bloquée. On bloque à son tour le fragment proximal grâce à une tige, un clou, ou une vis, de préférence, qui passe à travers l'os et au travers du tuyau qui comporte à ce niveau des orifices pour permettre le passage de ladite tige.

On voit donc bien les avantages selon la présente invention qui sont la simplicité du dispositif puisque seules deux pièces sont nécessaires et suffisantes pour un blocage parfait. De plus, le temps opératoire est considérablement réduit puisque, à part le percement du canal médullaire, il est nécessaire de ne percer qu'un seul trou pour la pénétration de la tige et que pour la mise en place définitive, il suffit de retirer le guide et d'insérer ladite tige pour obtenir l'effet recherché.

L'invention sera mieux comprise si l'on se réfère aux dessins annexés sur lesquels :
- la figure 1 montre en coupe un tuyau intra-médullaire selon l'invention, vu de face ;
- la figure 2 est une représentation de la partie distale du tuyau en conformation d'introduction dans le canal médullaire, lamelles rapprochées ;
- la figure 3 représente la coupe d'une lamelle suivant III-III′ de la figure 1 ;
- la figure 4 représente la coupe d'une lamelle suivant IV-IV′ de la figure 1, au niveau d'un bossage.

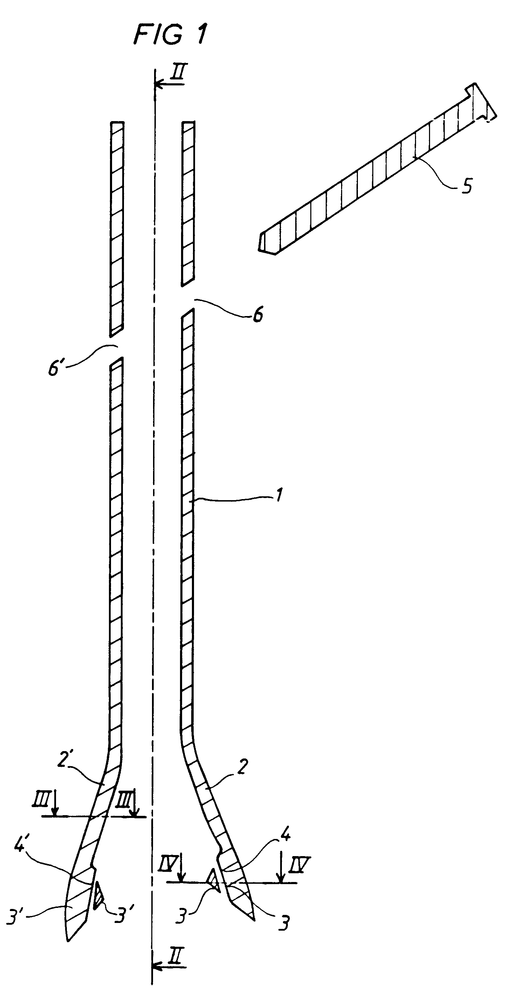

La figure 1 montre la section sensiblement cylindrique d'un tuyau 1 avec les lamelles spontanément écartées 2-2′ comportant des bossages 3-3′ décalés l'un par rapport à l'autre, percés en 4-4′ de manière à permettre le passage du guide et le blocage en position rapprochée.

Cette figure montre également la tige 5 perforant ledit tuyau en 6-6′ selon un angle d'environ 55° par rapport à l'axe longitudinal II-II′ dudit tuyau.

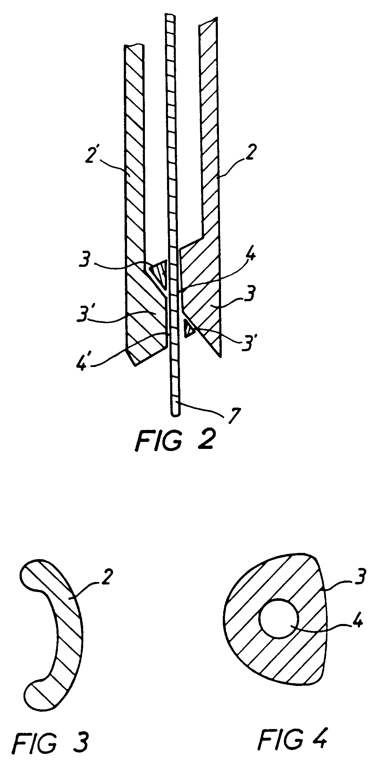

La figure 2 montre en particulier le guide 7 bloquant en position rapprochée les lamelles 2-2′ grâce à son insertion dans les orifices 4-4′ des bossages 3-3′.

La figure 3 représente une coupe de la lamelle suivant III-III′. On distingue en particulier les renflements que comportent dans cette réalisation les bords des lamelles.

La figure 4 représente la coupe suivant IV-IV′ d'un bossage et montre en particulier la perforation dans laquelle est introduit le guide en vue de l'introduction dans le canal médullaire.

## Revendications

1. Clou intra-médullaire pour empêcher la rotation dans les fractures d'os longs comportant un dispositif destiné à empêcher la rotation des fragments de l'os fracturé l'un par rapport à l'autre à sa partie distale et à sa partie proximale, constitué d'un tuyau présentant un passage longitudinal et un passage transversal pour une tige destinée à empêcher la rotation du côté proximal et deux lamelles (2,2') prévues à l'extrémité distale, caractérisé en ce que les lamelles sont élastiques, spontanément écartées l'une de l'autre, et munies à proximité de leur extrémité distale d'éléments (3,3') perforés d'un canal (4,4') selon l'axe du tuyau et disposés vers l'intérieur du tuyau, éléments dans lesquels en passant par le passage longitudinal on introduit pour l'installation dans l'os long un guide (7) rapprochant les lamelles, et libérant celles-ci, qui s'écartent, lorsque le guide (7) est retiré hors du clou.

2. Clou intra-médullaire selon la revendication 1, caractérisé en ce que l'élément (3,3') perforé d'un canal est un bossage (3,3') perforé d'un canal (4,4').

3. Clou intra-médullaire selon la revendication 1 ou 2, caractérisé en que la tige fait un angle d'environ 55° avec l'axe longitudinal du tuyau.

4. Clou intra-médullaire selon la revendication 3, caractérisé en ce que le passage de la tige est fileté et que la tige est une vis.

5. Clou intra-médullaire selon l'une quelconque des revendications 1 à 4, caractérisé en ce que le tuyau est fendu sur environ la moitié de sa longueur du côté distal de manière à constituer les lamelles (2,2').

## Claims

1. An intramedullary nail to prevent rotation in fractures of long bones comprising a device designed to prevent rotation of fragments of the fractured bone relative to one another at its distal part and at its proximal part, consisting of a pipe comprising a longitudinal passage and which comprises a transverse passage for a rod designed to prevent rotation on the proximal side and is equipped with two plates (2, 2') at the distal end, characterized in that the said blades are elastic, spontaneously spaced from one another, and equipped near their distal ends with elements (3, 3') perforated by a channel (4, 4') directed along the axis of the pipe and located towards the interior of the pipe, into which said elements, passing through the longitudinal passage, a guide (7) for the installation in the long bone is introduced which brings the blades close together and which releases the latter, which move away from one another, when the guide (7) is withdrawn out of the nail.

2. An intramedullary nail according to claim 1, characterized in that the element (3, 3') perforated by a channel is a boss (3, 3') perforated by a channel (4, 4').

3. An intramedullary nail according to claim 1 or claim 2, characterized in that the rod makes an angle of about 55° with the longitudinal axis of the pipe.

4. An intramedullary nail according to claim 3, characterized in that the passage for the rod is threaded and the rod is a screw.

5. An intramedullary nail according to any one of claims 1 to 4, characterized in that the distal part of the pipe is split over approximately half of its length so as to form the blades (2, 2').

## Patentansprüche

1. Intramedullärer Nagel zur Verhinderung des Verdrehens von Brüchen langer Knochen, mit einer Einrichtung, die dazu bestimmt ist, das Verdrehen von Fragmenten des gebrochenen Knochens bezüglich seines distalen Teils und seines proximalen Teils zueinander zu verhindern, und aus einem Rohr gebildet ist, das einen sich in Längsrichtung erstreckenden Durchlaß, einen in Querrichtung verlaufenden Durchlaß für einen Stift, der dazu bestimmt ist, das Verdrehen der proximalen Seite zu verhindern, und zwei am distalen Ende vorgesehene Lamellen (2, 2') aufweist, **dadurch gekennzeichnet**, daß die Lamellen elastisch sind, von selbst voneinander abstehen und benachbart zu ihren distalen Enden mit Elementen (3, 3') versehen sind, die von einem entsprechend der Achse des Rohres gerichteten Kanal (4, 4') durchbohrt und zum Rohrinneren hin angeordnet sind und in die zum Einsetzen in den langen Knochen unter Durchlaufen des sich in Längsrichtung erstreckenden Durchlasses eine Führung (7) eingeführt wird, die die Lamellen einander annähert und diese, sich voneinander entfernend, wieder frei gibt, wenn die Führung (7) aus dem Nagel wieder herausgezogen wird.

2. Intramedullärer Nagel nach Anspruch 1, dadurch gekennzeichnet, daß das von einem Kanal durchbohrte Element (3, 3') ein von einem Kanal (4,4') durchbohrter Vorsprung (3, 3') ist.

3. Intramedullärer Nagel nach Anspruch 1 oder 2, dadurch gekennzeichnet, daß der Stift mit der Längsachse des Rohres einen Winkel von ungefähr 55° ausbildet.

4. Intramedullärer Nagel nach Anspruch 3, dadurch gekennzeichnet, daß der Durchlaß für den Stift mit einem Gewinde versehen ist und daß der Stift eine Schraube ist.

5. Intramedullärer Nagel nach einem der Ansprüche 1 bis 4, dadurch gekennzeichnet, daß das Rohr auf ungefähr der Hälfte der Länge seiner distalen Seite so geschlitzt ist, daß sich die Lamellen (2, 2') ausbilden.
